# EUROPEAN PATENT SPECIFICATION

(11) **EP 2 637 649 B1**
(45) Date of publication and mention of the grant of the patent: **24.12.2014**
(21) Application number: 11781808.8
(22) Date of filing: 11.11.2011
(51) Int. Cl.: A61K 31/675, A61P 29/00, A61P 29/02, A61P 25/02

(54) **COMPOUNDS AND METHODS FOR TREATING PAIN**
VERBINDUNGEN UND VERFAHREN ZUR BEHANDLUNG VON SCHMERZ
COMPOSES ET PROCEDES DE TRAITEMENT DE LA DOULEUR

(30) Priority: 11.11.2010 EP 10190922
(43) Date of publication of application: 18.09.2013
(60) Divisional application: 13174939.2
(73) Proprietor: Akron Molecules AG, 1030 Wien (AT)
(72) Inventor: PENNINGER, Josef, A-1130 Vienna (AT); NEELY, Graham Gregory, Alexandria, Sydney, 2015 (AU); MCMANUS, Shane, A-1030 Vienna (AT); NILSSON, Henrik, 1030 Vienna (AT)
(74) Representative: Sonn & Partner Patentanwälte
(86) International application number: PCT/EP2011/069986
(87) International publication number: WO 2012/062925

(56) References cited:
- EP-A1- 1 685 849
- WO-A1-2011/127586
- WO-A1-2011/143607
- GILLE J ET AL: "[Acute pain management in proximal femoral fractures: femoral nerve block (catheter technique) vs. systemic pain therapy using a clinic internal organisation model].", DER ANAESTHESIST, vol. 55, no. 4, April 2006 (2006-04), pages 414-422, XP002668308, ISSN: 1432-055X
- JHAVERI MALHAR A ET AL: "Tenofovir-associated severe bone pain: I cannot walk!", JOURNAL OF THE INTERNATIONAL ASSOCIATION OF PHYSICIANS IN AIDS CARE (CHICAGO, ILL. : 2002), vol. 9, no. 5, September 2010 (2010-09), pages 328-334, XP009155922, ISSN: 1545-1097
- PERROT S ET AL: "Bone pain due to fractures revealing osteomalacia related to tenofovir-induced proximal renal tubular dysfunction in a human immunodeficiency virus-infected patient", JOURNAL OF CLINICAL RHEUMATOLOGY, vol. 15, no. 2, March 2009 (2009-03), pages 72-74, XP009155910, LIPPINCOTT WILLIAMS AND WILKINS USA ISSN: 1076-1608, DOI: 10.1097/RHU.0B013E31819C20D8
- NOEL J KAY ET AL: "Systematic review to establish the safety profiles for direct and indirect inhibitors of p38 Mitogen-activated protein kinases for treatment of cancer. A systematic review of the literature.", MEDICAL ONCOLOGY (NORTHWOOD, LONDON, ENGLAND), vol. 25, no. 3, 2008, pages 323-330, XP002674545, ISSN: 1357-0560
- WOZEL GOTTFRIED ET AL: "Cutaneous side effects of inhibition of VEGF signal transduction.", JOURNAL DER DEUTSCHEN DERMATOLOGISCHEN GESELLSCHAFT = JOURNAL OF THE GERMAN SOCIETY OF DERMATOLOGY : JDDG, vol. 8, no. 4, April 2010 (2010-04), pages 243-249, XP002674546, ISSN: 1610-0387
- KEATING G M ET AL: "Sorafenib: A review of its use in advanced hepatocellular carcinoma", DRUGS, vol. 69, no. 2, 1 January 2009 (2009-01-01), pages 223-240, XP009114085, ADIS INTERNATIONAL LTD, NZ ISSN: 0012-6667, DOI: 10.2165/00003495-200969020-00006
- JUNG YOUNG KUL ET AL: "Fanconi's Syndrome Associated with Prolonged Adefovir Dipivoxil Therapy in a Hepatitis B Virus Patient.", GUT AND LIVER, vol. 4, no. 3, September 2010 (2010-09), pages 389-393, XP002674547, ISSN: 1976-2283
- KWON SO YOUNG ET AL: "[A case of osteomalacia related to adefovir in a patient with chronic hepatitis B].", THE KOREAN JOURNAL OF GASTROENTEROLOGY = TAEHAN SOHWAGI HAKHOE CHI, vol. 56, no. 2, August 2010 (2010-08), pages 117-120, XP002674548, ISSN: 1598-9992
- CHAN HENRY L Y ET AL: "Treatment of hepatitis B e antigen positive chronic hepatitis with telbivudine or adefovir: a randomized trial.", ANNALS OF INTERNAL MEDICINE, vol. 147, no. 11, 4 December 2007 (2007-12-04), pages 745-754,W-228, XP002674549, ISSN: 1539-3704
- MAKI E ET AL: "Pharmacological studies of sulindac, a nonsteroidal antiinfalammatory agent (1). Anti-inflammatory, analgesic and antipyretic activities", EMBASE, XP002317644,

## Description

The present invention relates to the provision of pharmaceutical compounds suitable for the treatment of pain.

Acute and chronic pain affects millions of people after injury or surgery and those suffering from diseases like arthritis, cancer, and diabetes. Nociception (the detection of noxious or damaging stimuli) serves a crucial biological purpose: it alerts living organisms to environmental dangers, inducing the sensation of pain, reflex withdrawal and complex behavioural and emotional responses, which protect the organism from further damage. Noxious stimuli are detected by specialized high threshold primary sensory neurons (nociceptors), which transfer signals to the spinal cord and then transmit them to the brain for higher level processing that results in the conscious awareness of the sensation called pain. The functional importance of pain perception is exemplified by individuals with defects in nociception; patients with congenital insensitivity to pain do not survive past their twenties.

Two basic types of pain can be distinguished - acute and chronic. Acute or nociceptive pain is generally self-limiting and serves a protective biological function by warning of ongoing tissue damage caused by noxious chemical, thermal and mechanical stimuli. Examples of nociceptive pain include: postoperative pain, pain associated with trauma, and the pain associated with arthritis. Chronic pain, on the other hand, serves no protective biological function, and reflects either poor resolution of the painful stimuli, or is itself a disease process. Chronic pain is unrelenting and not self-limiting and can persist for years and even decades after the initial injury. Chronic pain is predominantly neuropathic in nature and may involve damage either to the peripheral or central nervous systems.

It is a goal of the present invention to provide means as defined in the claims of treating, ameliorating or suppressing pain, in particular by the use of novel compounds for this purpose.

The present invention therefore provides Remofovir (Pradefovir), Adefovir, especially Adefovir dipivoxil (Bis-POM PMEA) for use in the treatment or prevention of pain.

The inventive compound can be used in combination with other active analgesic/anti-pain compounds, preferably only with those described herein or above or in the claims, or used as single active analgesic/anti-pain compound.

Disclosed are compounds for use in the treatment or prevention of pain comprising a purine ring, such as N6-Benzyladenosine-5'-phosphate, V11294, LAS31025 ( Arofylline ), BRL-61063 ( Cimpyfylline ), Doxofylline, Etamiphylline, Etofyl-line, oxtriphyllin ( Choline theophyllinate ), Roscovitine, Tenofovir (PMPA) ( Tenofovir ), Remofovir (Pradefovir), Adefovir dipivoxil (Bis-POM PMEA) ( Adefovir ) and compounds comprising an ester group, such as Remofovir (Pradefovir), Adefovir dipivoxil (Bis-POM PMEA) ( Adefovir ).

The subject to be treated according to the present invention can be any non-human animal or a human. Preferably the subject is a mammal, in particular preferred embodiments a human.

According to the present invention pain and conditions associated with pain, e.g. itching or depression, can be treated or prevented, in particular in the meaning of a prophylactic administration. "Preventing" or "prevention" herein does not require absolute success in the sense of an absolute prevention of pain but indicates a reduced risk of developing a disease or painful condition, or developing pain with reduced severity. Likewise, "treatment" shall not be construed as an absolute cure, but may also relate to amelioration or suppression of pain or pain associated conditions.

Pain and pain associated conditions and diseases to be treated according to the present invention can include acute pain, chronic pain, somatogenic pain, neuropathic pain, psychogenic pain, heat induced pain, physical pain and nociception in general, or hyperalgesia. In particular embodiments the pain is selected from neuropathic pain, inflammatory pain, nociceptive pain, rheumatic pain, headache, low back pain, pelvic pain, myofascial pain, vascular pain, migraine, wound associated pain, inflammatory pain, arthritic pain, diabetic pain, pain from cancer or somatic visceral pain, all in both acute and chronic forms. The pain can also be related to phantom pain, pain from a part of the body that has been lost or from which the brain no longer receives physical signals.

Pain can be generally classified to two broad categories, acute and chronic. The treatment of any acute or chronic pain is subject matter of the present invention. Acute pain is usually associated with a specific cause such as a specific injury and is often sharp and severe. Acute pain begins suddenly and is not persistent. Chronic pain is long-term pain, with a typical duration of more than three months leading to significant psychological and emotional problems. Chronic pain is generally associated with clinical conditions characterised by chronic and/or degenerative lesions. Common examples of chronic pain are neuropathic pain (e.g. painful diabetic neuropathy, post-herpetic neuralgia), rheumatoid arthritis, osteoarthritis, fibromyalgia, back pain, headache, carpal tunnel syndrome, cancer pain, and chronic post-surgical pain. Pain can also be divided into a number of different subtypes according to differing pathophysiology, including nociceptive, inflammatory and neuropathic pain. Also some types of pain can be classified in multiple categories, for example pain associated with cancer can have a nociceptive and neuropathic component. Nociceptive pain consists of somatic pain (musculo-skeletal pain) and visceral pain (pain associated with the viscera, which encompass the organs of the abdominal cavity). Common causes of somatic pain include cancer metastasis such as to the bone and postsurgical pain from a surgical incision in addition to musculo-skeletal disorders such as dystrophinopathy, myalgia and polymyositis. Nociceptive pain also includes tissue injury-induced pain and inflammatory pain such as that associated with arthritis. Another type of inflammatory pain is visceral pain which includes pain associated with gastrointestinal disorders (GI) such as functional bowel disorder (FBD) and inflammatory bowel disease (IBD). Further examples of visceral pain include the pain associated with dysmenorrhea, cystitis and pancreatis and pelvic pain. Additional pain types include dysfunctional pain such as fibromyalgia, Temporomandibular Joint Disorder (TMJ), Irritable bowel syndrome (IBS) and musculo-skeletal pain. Neuropathic pain is caused by damage to the peripheral or central nervous system. Examples of central neuropathic pain include pain from spinal cord injury, multiple sclerosis, stroke and fibromyalgia. Diabetes and related metabolic disorders are a common cause of peripheral neuropathic pain (diabetic neuropathy). Some of the human conditions and pathologies characterised by the presence of neuropathic pain include, but are not limited to, cancer (cancer neuropathy), HIV neuropathy, Parkinson's disease, epilepsy, immunodeficiency, post-herpetic syndromes, trauma, ischaemia, sciatica, multiple sclerosis, peripheral neuropathy, trigeminal neuralgia, back pain, phantom limb pain, carpal tunnel syndrome, central poststroke pain and pain associated with chronic alcoholism, hypothyroidism, uraemia, spinal cord injury, and vitamin deficiency. Preferably the pain is neuropathic pain, such as trigeminal neuralgia, such as post-herpetic neuralgia, such as painful diabetic neuropathy, such as painful diabetic peripheral neuropathy, such as diabetic polyneuropathy, such as sciatic pain, such as radiculopathy, such as radicular pain or such as non-inflammatory neuropathic pain. Pain may be selected from fibromyalgia, postoperative pain, trigeminal neuralgia, post-herpetic neuralgia, painful diabetic neuropathy, painful diabetic peripheral neuropathy, diabetic polyneuropathy, sciatic pain, radiculopathy, radicular pain, lumbar pain. Preferably the pain is caused by the conditions as mentioned above related to the given pain type. In particular the pain type can be the only pain type in a subject. E.g. preferably a neuropathic pain is caused by affected nerves but not caused by inflammation, i.e. neuropathic pain is the only pain in the subject and is non-inflammatory.

"About" is used to refer to certain dosages that can vary from a given value, nevertheless with the same effects as the indicated dose. In some embodiments "about" may refer to +/- 20% or 10% of a given value.

Preferably the compound is administered in a dosage sufficient to treat or prevent pain or associated conditions and diseases. Administration can e.g. be a singe dose administration or a successive or repeated administration, e.g. twice a day, daily or in an interval of at least 1 day, at least 2 days, at least 3 days, at least 1 week, preferably at least 2 weeks, at least 4 weeks, at least 8 weeks or even more preferred at least 12 weeks. Preventive administrations are usually a short time before expected pain, if controllable or foreseeable - such as in scheduled surgery - e.g. up to 1hour (h), 2h, 3h, 4h, 5h, 6h, 8h, 10h, 12h or up to 24h or even up to 48h beforehand, as well as any interval in between.

According to a further preferred embodiment of the present invention, the compound is provided in a pharmaceutical composition or a medicament, in particular an analgesic. The composition or medicament may comprise a pharmaceutical carrier. Pharmaceutical carrier substances serve for a better tolerance of the medicament and allow for a better solubility as well as a better bioavailability of the active substances contained in the medicament. Examples of this are emulsifiers, thickening agents, redox components, starch, alcohol solutions, polyethylene glycol or lipids. The choice of a suitable pharmaceutical carrier is highly dependent on the manner of administration. For oral administrations, liquid or solid carriers may be used, for injections, liquid final compositions are required. For cellular targeting, such as for inhibitory nucleic acids, suitable vehicles can be included such as liposomes or microsomes.

Preferably, the medicament or the compound to be used according to the invention comprises buffer substances or tonic substances. By means of a buffer, the pH of the medicament can be adjusted to physiological conditions, and moreover, pH fluctuations can be attenuated, or buffered, respectively. An example thereof is a phosphate buffer. Tonic substances serve for adjusting the osmolarity and may comprise ionic substances, such as, e.g., inorganic salts, such as NaCl, or also non-ionic substances, such as, e.g. glycerol or carbohydrates.

The inventive compound or medicament can be administered topical, enteral or parenteral, in particular preferred oral or rectal, intravenous, intraarterial, intramuscular, subcutaneous, intradermal or intraperitoneal, transdermal, transmucosal or inhalational. Preferred routes of administration of the inventive agent according to the present invention are parenteral routes, preferably intraperitoneal or intravenous administration, intravenous administration being specifically preferred. Intravenous administration can be performed e.g. via bolus injection or by continuous intravenous delivery over a longer time period (e.g. 30 min to 6 h, especially 1 to 3 h). Further routes include oral or transdermal or subcutaneous routes. In particular preferred is oral administration. For digestible agents, such as active proteins, peptides or siRNA, parenteral routes are preferred.

The medicament or the compound to be used according to the invention can be prepared to be suitable for oral or intranasal administration. These administration forms of the medicament of the present invention allow for a rapid an uncomplicated uptake of the active substances via the mucous membranes. For a nasal intake, nose drops or nose sprays are suitable. For an oral administration, solid or liquid medicaments may, e.g., be taken directly or in a dissolved or diluted state, respectively.

The medicament or compound to be used according to the invention can be prepared for an intravenous, intra-arterial, intramuscular, intravascular, systemic, intraperitoneal or subcutaneous administration. For this purpose, e.g., injections or transfusions are suitable. Administrations directly into the bloodstream have the advantage that the active substances of the medicament will be distributed in the entire body and will quickly reach the target tissue or cells, in particular the peripheral nerves, spinal cord cells or brain cells.

The compound may be administered in a effective therapeutic dose. Effective doses are in the range of dosages known for the compounds for other, non-pain related administrations. In particular, for a specific use a dosage can be determined by a simple test using drosophila or mouse test systems. Further possible therapeutic doses of the compounds for the inventive treatment can be the same dosage disclosed or approved for other therapeutic uses for each of these compounds. Example dosages are at least 0.01 mg/kg, at least 0.1 mg/kg, at least 1 mg/kg, at least 10 mg/kg and/or up to 1 mg/kg, up to 10 mg/kg, up to 100 mg/kg, up to 1 g/kg, and any dosages in between. Preferred dosage ranges are between 0.01 mg/kg and 1 g/kg, preferably between 0.1 mg/kg and 100 mg/kg.

The examples show that the inventive pain tests revealed pharmaceutical compounds that are well known to be therapeutically applicable for the treatment of human conditions and diseases. The compounds may now also be used for the treatment of pain and pain associated secondary diseases.

The inventive administration can be for treatment, alleviation or prevention of pain or hyperalgesia in a subject.

The present invention is further illustrated by the following figures and examples.

### Figures:

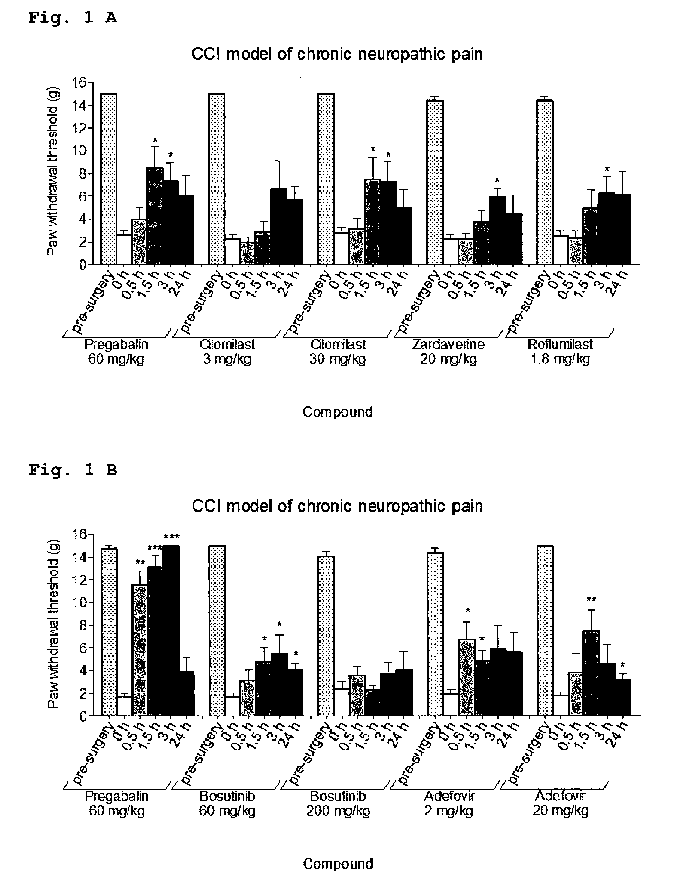

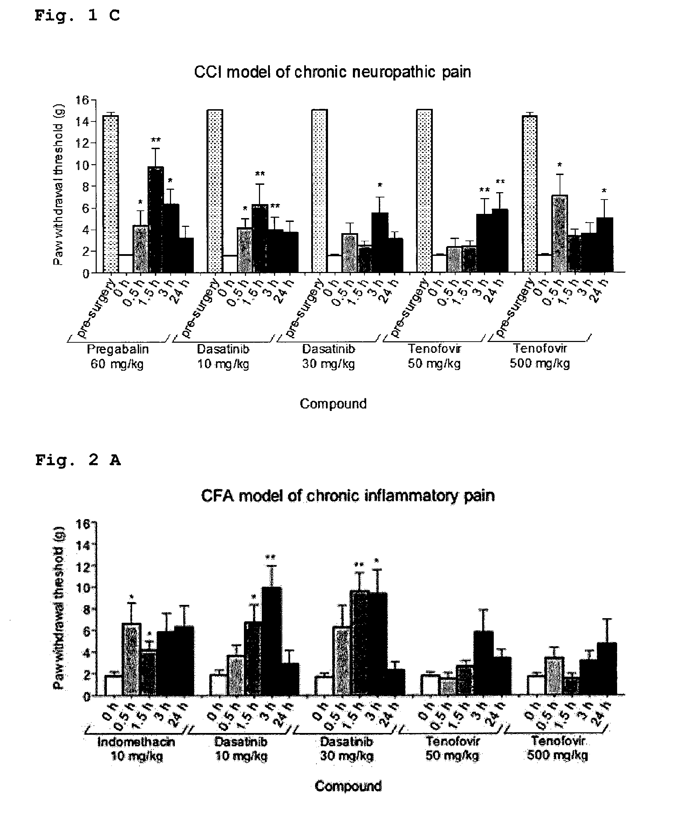

### Figure 1. Testing of anti-pain compounds

A: Compounds testing performed on pregabalin, cilomilast, zardaverine and roflumilast as described in Example 1.1.3.2. Data are presented as mean values ± SEM, n=8-10. Significantly different by Mann-Whitney U test compared to T=0 paw withdrawal thresholds (* p<0.05, ** p<0.01, *** p<0.001).
B: Compounds testing performed on pregabalin, bosutinib and adefovir as described in Example 1.1.3.3. Data are presented as mean values ± SEM, n=8-10. Significantly different by Mann-Whitney U test compared to T=0 paw withdrawal thresholds (* p<0.05, ** p<0.01, *** p<0.001).
C: Compounds testing performed on pregabalin, dasatinib and tenofovir as described in Example 1.1.3.1. Data are presented as mean values ± SEM, n=8-10. Significantly different by Mann-Whitney U test compared to T=0 paw withdrawal thresholds (* p<0.05, ** p<0.01, *** p<0.001).

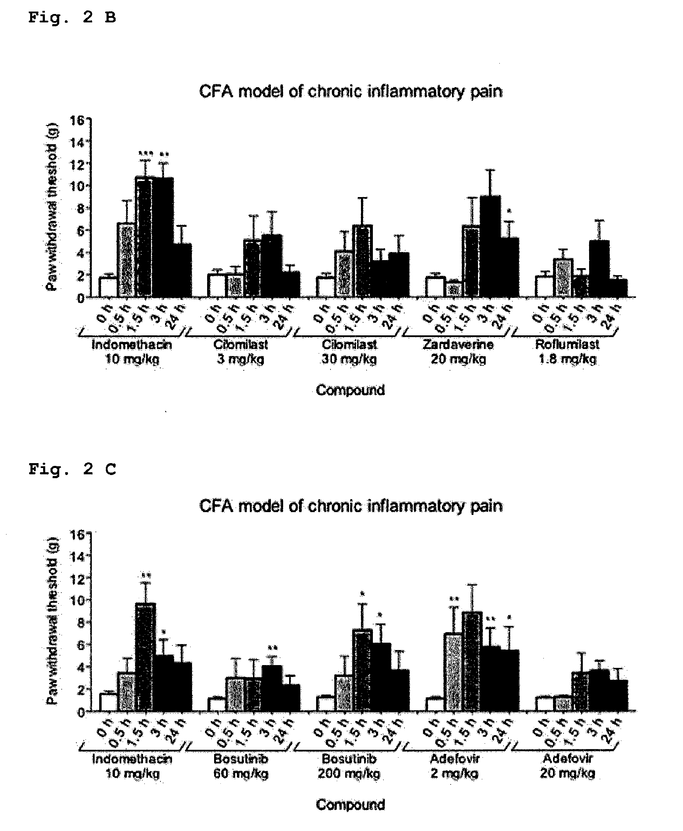

### Figure 2: Testing of anti-pain compounds, chronic inflammatory pain model

A: Von Frey's hairs assay for Indomethacin, Dasatinib, Tenofovir. Data are presented as mean values ± SEM, n=6-10. Significantly different by Mann-Whitney U test. * p<0.05, ** p<0.01, *** p<0.001 significantly different from post surgery withdrawal thresholds.
B: Von Frey's hairs assay for Indomethacin, Cimilast, Zardaverine, Roflumilast. Data are presented as mean values ± SEM, n=8-10. Significantly different by Mann-Whitney U test. * p<0.05, ** p<0.01, *** p<0.001 significantly different from post surgery withdrawal thresholds.
C: Von Frey's hairs assay for Indomethacin, Bosutinib, Adefovir. Data are presented as mean values ± SEM, n=8-10. Significantly different by Mann-Whitney U test. * p<0.05, ** p<0.01, *** p<0.001 significantly different from post surgery withdrawal thresholds.

### Examples:

### Example 1: Testing of anti-pain compounds

The basic principle of animal models of human pain involve the induction of a pain-like state in the organism resulting in characteristic behavioural and physical responses, such as hypersensitivity to touch (mechanical allodynia) and temperature (cold allodynia). The assessment of the efficacy of potential analgesics is determined based on said compound's ability to attenuate/ameliorate these symptoms.

### Example 1.1: Assessing chronic pain animal models - Chronic Constriction Injury

The Bennett and Xie chronic constriction injury (CCI) model is a model of mononeuropathic pain (Bennett and Xie, 1988). Rodents are subjected to a surgical procedure where gut ligatures are tied loosely around the sciatic nerve at the mid-thigh level. Symptoms of neuropathy develop in the operated paw over the following days including tactile allodynia and cold allodynia which are measured using Von Frey's hairs and paw withdrawal from a cold plate, respectively. Operated animals also exhibit other symptoms of spontaneous pain including thermal hyperalgesia , ectopic and spontaneous firing of sensory afferents, autotomy, licking and guarding of paw and sleep architecture abnormalities (Blackburn-Munro and Erichsen, 2005). Furthermore, electrophysiological studies have demonstrated the presence of both sensory nerve hyperexcitability and central sensitisation (wind up) in the dorsal horn and elsewhere (Blackburn-Munro and Erichsen, 2005). The Bennett and Xie model is thought to have predictive validity as an 88% concordance has been observed between activity in the model (any endpoint) and clinical efficacy in neuropathic pain (Kontinen and Meert 2003). Multiple drug classes including NSAIDs are ineffective in the clinical treatment of neuropathic pain and fail to ameliorate symptoms in the Bennett model despite the presence of an inflammation in the initial phase after surgery (eg Schafers et al., 2004; Takahashi et al., 2004, LaBuda and Little, 2005). Pregabalin, a drug approved for the treatment of various types of (chronic) pain, including neurophathic pain associated with diabetic peripheral neuropahty in humans, is effective at ameliorating symptoms of pain in the CCI model.

### Example 1.1.1. Test apparatus

Mechanical allodynia was assessed by Von Frey's hairs. These consist of a series of wires of progressive thickness each of which bend when a critical pressure (1.4, 2, 4, 6, 8, 10, 15 g) is applied. Animals were placed in floorless Perspex testing boxes resting on a wire mesh tray. Von Frey's hairs were then applied through the mesh floor to the sole of the left and right hind paws until either the animal sensed discomfort and moved the paw or the pressure applied to the Von Frey's hair exceeded the critical level and it was observed to bend. Von Frey's hairs were applied in ascending order until either a pain response (the paw was moved) was registered or the cut-off of 15 g was reached, using the Von Frey hair with the highest rating. Hairs were applied to each heel 8-10 times at a frequency of approximately 1 Hz. If a limb was moved in response to a probe, further testing was halted and the sensitivity to a mechanical stimulus was deemed to have been reached.

Sensitivity to cold (cold allodynia) was assessed by placing animal subjects on a cold plate held at 10°C. The latency (time) to lift the respective hind paw clear off the cold plate was measured. A cut-off of 180 s was applied.

### Example 1.1.2: Compound testing

This example demonstrates the effectiveness of compound(s) according to the invention in ameliorating mechanical and cold allodynia in rats with peripheral mononeuropathy induced by loose ligation of the sciatic nerve as described in Bennett and Xie, 1988.

The test apparatus according to Example 1.1.1 was used for assessing mechanical and thermal sensitivities.

Male Sprague-Dawley rats were habituated to the mechanical test apparatus (Von Frey's hairs, described in Example 1.1.1) during 5-10 min periods spread over two days and once to the cold plate set at 10°C for 3 minutes. Following habituation animals underwent a surgical procedure requiring anaesthetization under isoflurane, shaving of the left (ipsilateral) hind limb and swabbing with antiseptic followed by administration of sodium pentobarbitone. An incision was made to reveal the left sciatic nerve which was tied off with four loose ligatures of chromic cat gut. The exposed muscle was sutured with non-absorbable silk and the wound closed with surgical clips.

The animals were monitored in the hours post surgery and on a daily basis thereafter. Animals showing signs of ill health or autotomy were removed from the study. Approximately 12 and 18 days after surgery the animals were reassessed for sensitivity to Von Frey's hairs and to the cold plate.

Animal subjects meeting pre-defined criteria for hypersensitivity to mechanical (ipsilateral hind-paw moved at a pressure of ≤4 g) and thermal stimuli (≤78s withdrawal latency) in the operated (ipsilateral) hind-paw were allocated according to baseline mechanical and cold sensitivity scores to produce balanced treatment groups of 8 to 10 animals in each group.

### Example 1.1.3. Single dose/acute dosing

Each group of animals according to Example 1.1.2 was administered a single dose of either a positive control drug (pregabalin formulated in a vehicle of 0.5% methyl cellulose to a concentration of 12 mg/mL and administered po in a 5 mL/kg dosing volume to give a dose of 60 mg/kg) or a compound according to the invention. Animal subjects were reassessed for mechanical and cold allodynia 30 min, 90 min, 180 min and 24 hours after administration of the respective compound, using Von Frey's hairs and a cold plate set at 10°C. The cold plate assessment was performed 5 mins after each Von Frey test.

### Comparative Example 1.1.3.1 Test with dasatinib and tenofovir

The procedure as described in Example 1.1.2 was performed with the compounds dasatinib and tenofovir. In total, five groups of animals were included: one group of 10 animals received the positivie control drug, pregabalin, at 60 mg/kg and four groups of 8 animals each received either 10 mg/kg dasatinib, 30 mg/kg dasatinib, 50 mg/kg tenofovir or 500 mg/kg tenofovir, respectively (Fig. 1C). Dasatinib was formulated in a vehicle (50% PEG:50% water) to concentrations of 2 and 6 mg/mL and administered orally in a 5 mL/kg dosing volume to give doses of 10 mg/kg or 30 mg/kg. Tenofovir was formulated in a vehicle (0.5% methyl cellulose) to concentrations of 10 and 100 mg/mL and administered orally in a 5 mL/kg dosing volume to give doses of 50 and 500 mg/kg.

### Comparative Example 1.1.3.2 Description of cilomilast, zardaverine and roflumilast

The procedure as described in Example 1.1.2. was performed with the compounds cilomilast, zardaverine and roflumilast. In total, five groups of animals were included: one group of 10 animals received the positivie control drug, pregabalin, at 60 mg/kg and four groups of 8 animals each received either 3 mg/kg cilomilast, 30 mg/kg cilomilast, 20 mg/kg zardaverine or 1.8 mg/kg roflumilast, respectively (Fig. 1A). Cilomilast was formulated in water to concentrations of 0.6 and 6 mg/mL and administered orally in a 5 mL/kg dosing volume to give doses of 3 and 30 mg/kg. Zardaverine was formulated in a vehicle of 0.5% methyl cellulose to a concentration of 4 mg/mL and administered orally in a 5 mL/kg dosing volume to give a dose of 20 mg/kg. Roflumilast was formulated in a vehicle of 0.5% methyl cellulose to a concentration of 0.36 mg/mL and administered orally in a 5 mL/kg dosing volume to give a dose of 1.8 mg/kg.

### Example 1.1.3.3: Test with bosutinib and adefovir

The procedure as described in Example 1.1.2. was performed with the compound according to the invention being adefovir, and with bosutinib. In total, five groups of animals were included: one group of 10 animals received the positivie control drug, pregabalin, at 60 mg/kg and four groups of 8 animals each received either 60 mg/kg bosutinib, 200 mg/kg bosutinib, 2 mg/kg adefovir or 20 mg/kg adefovir, respectively (Fig. 1B). Bosutinib was formulated in 0.5% methyl cellulose to concentrations of 12 and 40 mg/mL and administered orally in a 5 mL/kg dosing volume to give doses of 60 and 200 mg/kg. Adefovir was formulated in 0.5% methyl cellulose to concentrations of 0.4 and 4 mg/mL and administered orally in a 5 mL/kg dosing volume to give doses of 2 and 20 mg/kg.

### Example 1.1.4: Chronic studies

Each group of animals according to Example 1.1.2 was administered either a positive control drug (pregabalin formulated in a vehicle of 0.5% methyl cellulose to a concentration of 12 mg/mL and administered po in a 5 mL/kg dosing volume to give an effective dose (e.g. 60 mg/kg)) or a compound according to the invention. Dosing was carried out one or several times daily for two days to eight weeks. Animal subjects were reassessed for mechanical and cold allodynia either daily, weekly, biweekly, monthly or at the end of the study at one or several time points: 30 min, 90 min, 180 min, 12 hours, 24 hours, 48 hours, 72 hours and 1 week after administration of the respective compound, using Von Frey's hairs and a cold plate set at 10°C.

### Example 1.2: Assessing inflammatory pain animal models - Complete Freunds Adjuvant (CFA)

Complete Freunds Adjuvant (CFA) consists of heat-killed mycobacterium suspended in mineral oil. When injected systemically into rodents, an immune response is triggered resulting in chronic inflammation of many organs such as skin, liver, spleen, eyes, bone marrow and particularly peripheral joints. The inflammatory response results in bone resorption and periosteal bone proliferation. Importantly this inflammatory state results in spontaneous pain and ectopic nerve cell firing. Thus, the resultant adjuvant disease exhibits polyarthritic symptoms.

When CFA is injected unilaterally into the limbs it elicits a monoarthritic-like condition and is thus used to model chronic inflammatory conditions. Unilateral injection allows the analysis of ipsilateral and contralateral effects of localised joint pain (Millan et al., 1988). Injection of CFA results in oedema of the affected joint, mechanical allodynia and mechanical and thermal hyperalgesia (Butler et al., 1992; Hsieh et al., 2010; Meotti et al., 2006; Staton et al., 2007). As these features resemble the clinical pathology of rheumatoid arthritis, CFA-induced chronic inflammation has been widely used as a model of this condition.

A wide variety of treatments acting via different mechanisms that have generated positive data in rat adjuvant-induced arthritic models have proven effective in subsequent clinical trials for rheumatoid arthritis (Hegen et al., 2008). Indeed, an analysis by Whiteside (Whiteside et al., 2008), concluded that activity in the rat CFA test could be used to predict the exposure needed for clinical efficacy. Both morphine-like opioids, steroids and NSAID analgesics can attenuate inflammation-associated allodynia/hyperalgesia and normalise nociceptive sensitivity (Jett et al., 1999, da Silva Filho et al., 2004).

### Example 1.2.1: Test system

This example demonstrates the effectiveness of a compound according to the invention in ameliorating mechanical and cold allodynia in rats with inflammatory pain induced by injection of an adjuvant (CFA) into the limbs.

The test apparatus according to Example 1.1.1 was used for assessing mechanical and thermal sensitivities.

53 male Sprague-Dawley rats were habituated to the test apparatus and tested for basal sensitivity to mechanical and thermal stimuli in both the right (ipsilateral) and left (contralateral) paws using Von Freys hair and a radiant heat source, respectively, on the day prior to Complete Freunds Adjuvant (CFA) administration (Day 0). The following morning (Day 1), animals were subjected to a subplantar injection of 50% complete CFA in a 100uL injection volume into the ipsilateral hind-paw. Two days later (Day 3), baseline mechanical and thermal sensitivities were reassessed in the CFA-injected (ipsilateral) and non-injected (contralateral) hind-paws. 42 rats meeting pre-defined criteria for hypersensitivity to mechanical (ipsilateral hind-paw moved at a pressure of <4g) and thermal stimuli (>30% difference in latency time between ipsi- and contra-lateral hind-paws) were assigned to treatment groups of 8 to 10 animals in each group. One hour later, at T=0, drug administration commenced.

### Example 1.2.2 Acute/single dosing

One group of animals according to Example 1.2.1 was administered a single dose of the drug indomethacin (positive control), an NSAID with demonstrated efficacy in the CFA model and in human inflammatory/arthritic diseases. Indomethacin was formulated in 50% 0.1 M Na₂CO₃; 47.5% phosphate buffered saline (PBS): taken to pH 7 with 2.5% 1M HCl at a concentration of 2 mg/mL to give a dose of 10 mg/kg when administered intraperitoneally in a 5 mL/kg injection volume. The remaining groups of animals were administered a single dose of compound(s) according to the invention. 30, 90, 180 min and 24h post-dose, all groups of rats were re-assessed for mechanical allodynia and thermal hyperalgesia in both the treated and untreated hind-paws, using Von Frey's hairs and a cold plate set at 10°C in accordance with Example 0. All readings were compared to the basal sensitivity reading (T=0) of the (ipsilateral) paw in each animal.

### Comparative Example 1.2.2.1 Test with dasatinib and tenofovir

The procedure as described in Example 1.2.1 was performed with the compounds dasatinib and tenofovir. In total, five groups of animals were included: one group of 10 animals received the positivie control drug, indomethacin, intraperitonally at a dose of 10 mg/kg Four groups of 8 animals each received either 10 mg/kg dasatinib, 30 mg/kg dasatinib, 50 mg/kg tenofovir or 500 mg/kg tenofovir, respectively (Fig. 2A). Dasatinib was formulated in a vehicle (50% PEG:50% water) to concentrations of 2 and 6 mg/mL and administered orally in a 5 mL/kg dosing volume to give doses of 10 and 30 mg/kg. Tenofovir was formulated in a vehicle (0.5% methyl cellulose) to concentrations of 10 and 100 mg/mL and administered orally in a 5 mL/kg dosing volume to give doses of 50 and 500 mg/kg.

### Comparative Example 1.2.2.2 Test with cilomilast, zardaverine and roflumilast

The procedure as described in Example 1.2.1 was performed with the compounds cilomilast, zardaverine and roflumilast. In total, five groups of animals were included: one group of 10 animals received the positivie control drug, indomethacin, at 10 mg/kg and four groups of 8 animals each received either 3 mg/kg cilomilast, 30 mg/kg cilomilast, 20 mg/kg zardaverine or 1.8 mg/kg roflumilast, respectively (Fig. 2B). Cilomilast was formulated in water to concentrations of 0.6 and 6 mg/mL and administered orally in a 5 mL/kg dosing volume to give doses of 3 and 30 mg/kg. Zardaverine was formulated in a vehicle of 0.5% methyl cellulose to a concentration of 4 mg/mL and administered orally in a 5 mL/kg dosing volume to give doses of 20 mg/kg. Roflumilast was formulated in a vehicle of 0.5% methyl cellulose to a concentrations of 0.36 mg/mL and administered orally in a 5 mL/kg dosing volume to give doses of 1.8 mg/kg.

### Example 1.2.2.3 Test with bosutinib and adefovir

The procedure as described in Example 1.2.1 was performed with the compounds according to the invention being adefovir, and with bosutinib. In total, five groups of animals were included: one group of 10 animals received the positivie control drug, indomethacin, at 10 mg/kg and four groups of 8 animals each received either 60 mg/kg bosutinib, 200 mg/kg bosutinib, 2 mg/kg adefovir or 20 mg/kg adefovir, respectively (Fig. 2C). Bosutinib was formulated in 0.5% methyl cellulose to concentrations of 12 and 40 mg/mL and administered orally in a 5 mL/kg dosing volume to give doses of 60 and 200 mg/kg. Adefovir was formulated in 0.5% methyl cellulose to concentrations of 0.4 and 4 mg/mL and administered orally in a 5 mL/kg dosing volume to give doses of 2 and 20 mg/kg.

### Example 1.2.3. Chronic studies

Each group of animals according to Example 1.2 was administered either a positive control drug (indomethacin, which was formulated in 50% 0.1 M Na₂CO₃; 47.5% phosphate buffered saline (PBS): taken to pH 7 with 2.5% 1M HCl at a concentration of 2 mg/mL to give an effective dose (e.g. 10 mg/kg when administered intraperitoneally in a 5 mL/kg injection volume)), or a compound according to the invention. Dosing was carried out one or several times daily for two days to eight weeks. Animal subjects were reassessed for mechanical and cold allodynia either daily, weekly, biweekly, monthly or at the end of the study at one or several time points: 30 min, 90 min, 180 min, 12 hours, 24 hours, 48 hours, 72 hours and 1 week after administration of the respective compound, using Von Frey's hairs and a cold plate set at 10°C according to Example 0.

### Example 2. Clinical study

Patients with a long term or chronic pain condition are eligible for the study.

After initial screening, a total of 20 to 200 patients are randomized to one of two treatment groups. Patients in one treatment group receive a compound according to the invention at a pharmaceutically active dose and patients in the other (control) treatment group receive either placebo or an active control drug at a pharmaceutically active dose. A preferred active control drug is pregabalin. Preferably, the study is carried out in a double-blinded fashion.

Treatment duration is between 1 and 15 weeks, and efficacy evaluation is carried out as the average of the pain scores recorded for the past 1 to 7 days (preferably 7 days) relative to the day chosen for efficacy evaluation, comparing the group receiving the compound according to the invention with the control group. The pain scores are preferably assessed based on patients' daily pain diaries, in which they record their daily pain score on an 11-point (0 = "no pain" to 10 = "worst possible pain") numeric rating scale (NRS) [Arezzo et al., 2008].

The primary endpoint of the study is preferably a comparison of the average pain score for the last 7 available pain diary entries at the end of the treatment phase.

### Example 3. Painful diabetic peripheral neuropathy (PDPN)

A clinical study according to Example 2 is carried out with the following key inclusion criteria: patients are men or women ≥18 years of age with type 1 or type 2 diabetes with HbAlC ≤11% and painful diabetic peripheral neuropathy of at least 3 months' duration. Patients score at least 40 mm on the Short-Form McGill Pain Questionnaire (SF-MPQ) Visual Analog Scale (VAS) [Arezzo et al., 2008], or any other standard VAS. At randomization, patients have completed at least four daily pain diary entries (using an 11-point NRS) and should have an average daily pain score ≥4 over the past 7 days.

### Example 4. Post-herpetic neuralgia

A clinical study according to Example 2 is carried out with the following key inclusion criteria: patients are men or women ≥18 years of age with persistent pain for at least 6 months after the onset of herpes zoster rash. Patients score at least 40 mm on the Short-Form McGill Pain Questionnaire (SF-MPQ) Visual Analog Scale (VAS) [Arezzo et al., 2008], or any other VAS.

### Example 5. Mixed neuropathy

A clinical study according to Example 2 is carried out with patients selected according to inclusion criteria in Example 3 as well as Example 4.

### REFERENCES

Bennett G.J., Xie Y.K., (1988). Pain, 33, 87-107. Blackburn-Munro G, Erichsen H.K., (2005). Current Pharm Des, 11, 2961-2975.
Butler, S.H., Godefroy, F., Besson, J.-M., Weil-Fugazza, J., (1992). Pain, 48; 73-81.
Hegen, M., Keith, J.C., Collins, M., Nickerson-Nutter, C.L., (2008). Ann Rheum Dis, 67: 1505-1515.
Hsieh, G.C., Chandran, P., Salyers, A.K., Pai, M., Zhu, C.Z., Wensink, E.J., Witte, D.G., Miller, T.R., Mikusa, J.P., Baker, S.J., Wetter, J.M., Marsh, K.C., Hancock, A.A., Cowart, M.D., Esbenshade, T.A., Brioni, J.D., Honore, P., (2010). Pharmacol Biochem Behav, 95; 41-50.
Kontinen V.K., and Meert T.F., (2003). In: eds Dostrovsky J.O., Carr D.B., and Koltzenburg, M., Prog in Pain Res Management 24, pp489-498, IASP press, Seattle.
LaBuda, C.J., and Little, P., (2005) J Neurosci Meths, 144, 175-181.
Meotti, F.C., Missau, F.C., Ferreira, J., Pizzolatti, M.G., Mizuzaki, C., Nogueira, C.W., Santos, A.R.S., (2006). Biochem Pharmacol, 72; 1707-17134.
Millan, M.J., Czlonkowski, A., Morris, B., Stein, C., Arendt, R., Huber, A., et al., (1988). Pain, 35, 299-312.
Schafers, M., Marziniak, M., Sorkin, L.S., Yaksh, T.L., Sommer C., (2004) Exp Neurol ., 185, 160-168.
Staton, P.C., Wilson, A.W., Bountra, C., Chessell, I.P., Day, N.C., (2007). Eur J Pain' 11; 283-289.
Takahashi M., Kawaguchi, M., Shimada, K.,, Konishi, N., Furuya H., Nakashima, T., (2004). Neurosci Letts, 356, 37-40. Whiteside, G.T., Adedoyin, A., Leventhal, L., (2008). Neuropharmacology, 54, 767-775.
Jett, M.F., Ramesha, C.S., Brown, C.D., Chiu, S., Emmett, C., Voronin, T., Sun, T., O'Yang, C., Hunter, J.C., Eglen, R.M., Johnson, R.M., (1999). Characterization of the analgesic and anti-inflammatory activities of ketorolac and its enantiomers in the rat. J Pharmacol Exp Ther, 288(3):1288-97.
da Silva Filho A.A., Andrade e Silva M.L., Carvalho J.C., Bastos J.K., (2004). Evaluation of analgesic and anti-inflammatory activities of Nectandra megapotamica (Lauraceae) in mice and rats. J Pharm Pharmacol., 56(9):1179-84.
Arezzo, J.C., Rosenstock, J., LaMoreaux, L., Pauer, L., (2008), Efficacy and safetyof pregabalin 600 mg/d for treating painful diabetic peripheral neuropathy: a double-blind placebocontrolled trial. BMC Neurology, 16;8:33.

## Claims

1. Remofovir (Pradefovir) for use in the treatment or prevention of pain.

2. Adefovir for use in the treatment or prevention of pain.

3. Adefovir for use according to claim 2, **characterized in that** Adefovir is Adefovir dipivoxil (Bis-POM PMEA).

4. Compound for use according to any one of claims 1 to 3, **characterized in that** the compound is administered topically, enterally or parenterally, in particular preferred orally or rectally, intravenously, intraarterially, intramuscularly, subcutaneously, intradermally, intraperitoneally, transdermally, transmucosally or by inhalation.

5. Compound for use according to any one of claims 1 to 4, **characterized in that** the subject to be treated is a mammal, preferably a human.

6. Compound for use according to any one of claims 1 to 5, **characterized in that** the compound is provided in a medicament.

7. Compound for use according to any one of claims 1 to 6, **characterized in that** the compound is provided together with a pharmaceutically acceptable carrier or buffer.

8. Compound for use according to any one of claims 1 to 7, **characterized in that** the compound is administered in a therapeutically effective dosage, preferably a dosage of between 0.01 mg/kg and 1 g/kg.

9. Compound for use according to any one of claims 1 to 8, wherein pain is selected from or associated with chronic or acute pain or hyperalgesia pain, somatogenic pain, neuropathic pain, psychogenic pain, heat induced pain, physical pain, nociception, hyperalgesia, rheumatic pain, headache, low back pain, pelvic pain, myofascial pain, vascular pain, migraine, wound associated pain, inflammatory pain, arthritic pain, diabetic pain, pain from cancer, somatic visceral pain, fibromyalgia, postoperative pain, phantom pain, trigeminal neuralgia, post-herpetic neuralgia, painful diabetic neuropathy, painful diabetic peripheral neuropathy, diabetic polyneuropathy, sciatic pain, radiculopathy, radicular pain, lumbar pain or any combination thereof.

10. Compound for use according to claim 9, wherein pain is chronic pain.

11. Compound for use according to claim 9 or 10, wherein pain is neuropathic pain.

12. Compound for use according to claim 9, wherein pain is inflammatory pain.

13. Compound for use according to claim 9, 10 or 11, wherein pain is non-inflammatory pain.

## Patentansprüche

1. Remofovir (Pradefovir) zur Verwendung bei der Behandlung oder Prävention von Schmerz.

2. Adefovir zur Verwendung bei der Behandlung oder Prävention von Schmerz.

3. Adefovir zur Verwendung nach Anspruch 2, **dadurch gekennzeichnet, dass** Adefovir Adefovirdipivoxil (Bis-POM PMEA) ist.

4. Verbindung zur Verwendung nach einem der Ansprüche 1 bis 3, **dadurch gekennzeichnet, dass** die Verbindung topisch, enteral oder parenteral verabreicht wird, besonders bevorzugt oral oder rektal, intravenös, intraarteriell, intramuskulär, subkutan, intradermal, intraperitoneal, transdermal, transmukosal oder durch Inhalation.

5. Verbindung zur Verwendung nach einem der Ansprüche 1 bis 4, **dadurch gekennzeichnet, dass** das zu behandelnde Subjekt ein Säugetier ist, bevorzugt ein Mensch.

6. Verbindung zur Verwendung nach einem der Ansprüche 1 bis 5, **dadurch gekennzeichnet, dass** die Verbindung in einem Medikament vorgesehen ist.

7. Verbindung zur Verwendung nach einem der Ansprüche 1 bis 6, **dadurch gekennzeichnet, dass** die Verbindung zusammen mit einem pharmazeutisch annehmbaren Träger oder Puffer vorgesehen ist.

8. Verbindung zur Verwendung nach einem der Ansprüche 1 bis 7, **dadurch gekennzeichnet, dass** die Verbindung in einer therapeutisch wirksamen Dosis verabreicht wird, bevorzugt in einer Dosis zwischen 0,01 mg/kg und 1 g/kg.

9. Verbindung zur Verwendung nach einem der Ansprüche 1 bis 8, wobei Schmerz ausgewählt ist aus, oder in Zusammenhang steht mit, chronischem oder akutem Schmerz oder Hyperalgesieschmerz, somatogenem Schmerz, neuropathischem Schmerz, psychogenem Schmerz, hitzeinduziertem Schmerz, körperlichem Schmerz, Nozizeption, Hyperalgesie, rheumatischem Schmerz, Kopfschmerzen, Schmerzen im unteren Rücken, Schmerzen im Beckenbereich, myofaszialem Schmerz, vaskulärem Schmerz, Migräne, mit Wunden zusammenhängendem Schmerz, Entzündungsschmerz, arthritischem Schmerz, diabetischem Schmerz, durch Krebs verursachtem Schmerz, somatisch viszeralem Schmerz, Fibromyalgie, postoperativem Schmerz, Phantomschmerz, Trigeminusneuralgie, postherpetischer Neuralgie, schmerzvoller diabetischer Neuropathie, schmerzvoller diabetischer peripheraler Neuropathie, diabetischer Polyneuropathie, Ischiasschmerz, Radikulopathie, Wurzelschmerz, Lendenschmerz, oder eine beliebige Kombination dieser.

10. Verbindung zur Verwendung nach Anspruch 9, wobei Schmerz chronischer Schmerz ist.

11. Verbindung zur Verwendung nach Anspruch 9 oder 10, wobei Schmerz neuropathischer Schmerz ist.

12. Verbindung zur Verwendung nach Anspruch 9, wobei Schmerz Entzündungsschmerz ist.

13. Verbindung zur Verwendung nach Anspruch 9, 10 oder 11, wobei Schmerz nicht-Entzündungsschmerz ist.

## Revendications

1. Rémofovir (Pradéfovir) pour une utilisation dans le traitement ou la prévention de la douleur.

2. Adéfovir pour une utilisation dans le traitement ou la prévention de la douleur.

3. Adéfovir pour une utilisation suivant la revendication 2, **caractérisé en ce que** l'Adéfovir est l'Adéfovir-dipivoxil (Bi-POM PMEA).

4. Composé pour une utilisation suivant l'une quelconque des revendications 1 à 3, **caractérisé en ce qu'**il est administré par voie topique, entérale ou parentérale, de manière particulièrement appréciée par voie orale ou rectale, intraveineuse, intra-artérielle, intramusculaire, sous-cutanée, intradermique, intrapéritonéale, transdermique ou transmuqueuse ou bien par inhalation.

5. Composé pour une utilisation suivant l'une quelconque des revendications 1 à 4, **caractérisé en ce que** le sujet à traiter est un mammifère, de préférence un être humain.

6. Composé pour une utilisation suivant l'une quelconque des revendications 1 à 5, **caractérisé en ce qu'**il est fourni dans un médicament.

7. Composé pour une utilisation suivant l'une quelconque des revendications 1 à 6, **caractérisé en ce qu'**il est fourni conjointement avec un support ou tampon pharmaceutiquement acceptable.

8. Composé pour une utilisation suivant l'une quelconque des revendications 1 à 7, **caractérisé en ce qu'**il est administré à une posologie thérapeutiquement efficace, de préférence une posologie entre 0,01 mg/kg et 1 g/kg.

9. Composé pour une utilisation suivant l'une quelconque des revendications 1 à 8, la douleur étant choisie dans ou associée à la douleur chronique ou aiguë ou la douleur hyperalgésique, la douleur somatogène, la douleur neuropathique, la douleur psychogène, la douleur induite par la chaleur, la douleur physique, la nociception, l'hyperalgésie, la douleur rhumatismale, les céphalées, la douleur dans le bas du dos, la douleur pelvienne, la douleur myofasciale, la douleur vasculaire, la migraine, la douleur associée à une plaie, la douleur inflammatoire, la douleur arthritique, la douleur diabétique, la douleur due à un cancer, la douleur viscérale somatique, la fibromyalgie, la douleur postopératoire, la douleur des amputés, les névralgies du trijumeau, les névralgies post-herpétiques, la neuropathie diabétique douloureuse, la neuropathie périphérique diabétique douloureuse, la polyneuropathie diabétique, la douleur due à la sciatique, la radiculopathie, la douleur radiculaire, la douleur lombaire ou n'importe laquelle de leurs associations.

10. Composé pour une utilisation suivant la revendication 9, la douleur étant la douleur chronique.

11. Composé pour une utilisation suivant la revendication 9 ou 10, la douleur étant la douleur neuropathique.

12. Composé pour une utilisation suivant la revendication 9, la douleur étant la douleur inflammatoire.

13. Composé pour une utilisation suivant la revendication 9, 10 ou 11, la douleur étant une douleur non-inflammatoire.
